# EUROPÄISCHE PATENTSCHRIFT

(11) **EP 0 478 838 B1**
(45) Veröffentlichungstag und Bekanntmachung des Hinweises auf die Patenterteilung: **02.08.1995**
(21) Anmeldenummer: 90119087.6
(22) Anmeldetag: 05.10.1990
(51) Int. Cl.: A61K 31/19, A61K 47/18

(54) **Ibuprofen- und S-(+)-Ibuprofen Zubereitungen und Verfahren zu ihrer Herstellung**
Compositions containing ibuprofen and S-(+)-ibuprofen, and process to prepare them
Compositions à base de ibuprofène et S-(+)-ibuprofène, et procédé de préparation

(43) Veröffentlichungstag der Anmeldung: 08.04.1992
(73) Patentinhaber: PHARMATRANS SANAQ AG, 4011 Basel (CH)
(72) Erfinder: Bauer, K., Prof.Dr.Chem., W-7800 Freiburg-Tiengen (DE)
(74) Vertreter: Kraus, Walter, Dr.

(56) Entgegenhaltungen:
- EP-A- 0 267 321
- EP-A- 0 299 668
- EP-A- 0 346 006

## Beschreibung

Die Erfindung betrifft eine Zubereitung mit verbesserter Tablettierbarkeit, verbesserter Festigkeit und erhöhtem Schmelzbereich, die Ibuprofen- oder das reine Enantiomer S-(+)-Ibuprofen sowie übliche Arzneimittel-Zusatzstoffe und/oder Arzneimittel-Trägerstoffe enthält, sowie ein Verfahren zu ihrer Herstellung.

Ibuprofen (INN) der folgenden Formel (I) ist ein Racemat aus den beiden Enantiomeren R-(-)- und S-(+)-2-(4-Isobutylphenyl)-propionsäure. 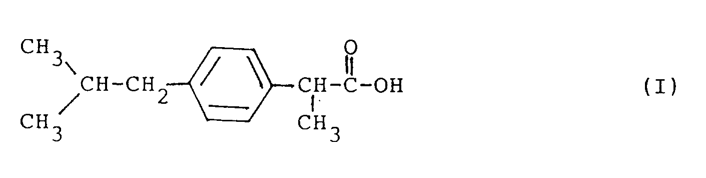
Die therapeutisch wirksame Form ist das S-(+)-Enantiomer, die S-(+)-2-(4-Isobutylphenyl)-propionsäure bzw. das S-(+)-Ibuprofen, während das R-(-)-Enantiomer, das R-(-)-Ibuprofen praktisch unwirksam ist. Da das Racemat im Organismus enzymatisch in die wirksame S-Form umgewandelt wird, ist es grundsätzlich nicht falsch, wenn das Stereoisomerengemisch als Arzneistoff eingesetzt wird. Das S-(+)-Ibuprofen ist jedoch besser oder rascher wirksam, so daß es in geringeren Dosen appliziert werden kann (vgl. A.J. Hutt und J. Caldwell; Review: The metabolic chiral inversion of 2-arylpropionic acids - a novel route with pharmacological consequences, J. PHARM. PHARMACOL. 35, 693 - 704 (1983)).

Ibuprofen und S-(+)-Ibuprofen werden in der Therapie als nichtsteroide Antirheumatika, Antiarthritika oder Analgetika eingesetzt.

Aus technologischer Sicht besitzen diese beiden Arzneistoffe folgende Nachteile. Sie sind schlecht in Wasser löslich und haben relativ niedrige Schmelztemperaturen. Ibuprofen schmilzt bei 75 bis 77°C und S-(+)-Ibuprofen sogar noch tiefer bei etwa 48 bis 51°C. Stoffe mit niedrigen Schmelzpunkten führen bekannterweise bei der Tablettenkompression infolge von Sintervorgängen und durch Kleben an den Stempeln und Matrizen der Tablettenpressen zu mehr oder weniger stark störenden Produktionsproblemen (H. Sucker, P. Fuchs und P. Speiser: Pharmazeutische Technologie, Georg Thieme Verlag, Stuttgart 1978, S. 381).

Das Kleben dieser niedrigschmelzenden Arzneistoffe kann durch Überdosen von Antiklebmitteln, (Formentrennmitteln) behoben werden. Dabei werden die Mischungen jedoch hydrophobiert. Daraus resultieren wiederum schlechte Bioverfügbarkeiten, oder die Tabletten werden infolge Überdosierung der Antiklebmittel zu weich.

Bis zu einem bestimmten Grad lassen sich daher die Komplikationen bei der Tablettenherstellung durch die Zugabe von bestimmten Tabletten-Hilfsstoffen, wie Antiklebmittel bzw. Schmiermittel, in höheren Dosen sowie durch eine drastische Reduktion der Preßgeschwindigkeit beheben. Darüber hinaus ist erfahrungsgemäß bei Tabletten, die niedrigschmelzende Ingredienten enthalten, nach bestimmten Lagerzeiten infolge von Sinterungen mit Nachhärtungen zu rechnen (K.H. Bauer, K.H. Frömming und C. Führer: Pharmazeutische Technologie, Georg Thieme Verlag, Stuttgart, New York, 1986). Diese Nachhärtungen ziehen Verschlechterungen der Zerfallszeit und als Folge davon nicht ausreichende Arzneistofffreisetzungen bzw. nicht ausreichende Bioverfügbarkeiten nach sich.

Bisher wurde mit wechselndem Erfolg versucht, durch Zusatz von Formentrenn- oder Schmiermitteln, Trockenmitteln (zum Beispiel hochdisperse Kieselsäure), geeigneten Füllmitteln und von starken Zerfallsbeschleunigern (zum Beispiel quervernetztem PVP), vor allem jedoch durch überdurchschnittliche Erhöhungen der Zusatzmengen dieser Hilfsstoffe, diese Nachteile zu beheben.

In der EP-A-0 267 321 ist ein Ibuprofen enthaltendes Arzneimittel beschrieben, welches Ibuprofen nur in der (S)-(+)-Form enthält. Diees bekannte Arzneimittel besitzt jedoch die oben genannten Nachteile, d.h. es läßt sich nur schlecht tablettieren.

Das Zusammensintern oder -schmelzen kann durch Zusatz von größeren Mengen Füllmitteln, zum Beispiel Cellulosepulver oder Lactose, oder durch Sprengmittel, zum Beispiel Stärke, vermindert werden. Zu hohe Zusätze von Sprengmittel haben jedoch meist zu weiche Tabletten zur Folge, da sich Stärke oder andere ähnlich elastische Stoffe allein oder in hoher Dosierung nur schlecht komprimieren läßt, oder die Tabletten werden so groß, daß sie schwer zu schlucken sind.

Der vorliegenden Erfindung liegt die Aufgabe zugrunde, eine Ibuprofen- oder S-(+)-Ibuprofen enthaltende Zubereitung mit verbesserter Tablettierbarkeit, verbesserter Festigkeit und erhöhtem Schmelzpunkt und ein Verfahren zu ihrer Herstellung zur Verfügung zu stellen. Die Tabletten sollen eine solche Größe aufweisen, daß sie leicht zu schlucken sind. Insbesondere soll eine Zubereitung zur Verfügung gestellt werden, welche sich leicht zu Tabletten verarbeiten läßt und die nicht an den Stempeln und Matrizen der Tablettenpressen klebt, so daß bei der Herstellung von Tabletten keine Produktionsprobleme auftreten. Die Zubereitung wie auch die daraus hergestellten Arzneimittel, wie Tabletten, Kapseln usw., sollen ausreichende Lagerzeiten aufweisen. Erfindungsgemäß soll eine Zubereitung zur Verfügung gestellt werden, die eine ausreichende Bioverfügbarkeit des Wirkstoffs auch nach einer bestimmten Lagerzeit gewährleistet.

Es wurde nun gefunden, daß die vorher geschilderten Probleme bei der Herstellung von Tablettengranulaten auf einfache Weise vermieden werden können, wenn Ibuprofen oder S-(+)-ibuprofen ganz oder teilweise in ihre Calciumsalze überführt werden und diese zur Tablettenherstellung eingesetzt werden. Bereits 25% Calciumsalz des Ibuprofens verbessern die Tablettiereigenschaften merklich; bevorzugt werden 50 bis 100%, d.h., wenn die Hälfte bis die Gesamtdosis Ibuprofen als Calciumsalz vorliegt. Es ist weiterhin bevorzugt, daß bei der Durchführung der üblichen Mischer- oder Wirbelschicht-Granulierverfahren Alkali- oder Ammoniumhydroxid- oder -salz-Lösungen als Granulierflüssigkeit verwendet werden.

Gegenstand der Erfindung ist eine Zubereitung mit verbesserter Tablettierbarkeit, die Ibuprofen und/oder S-(+)-Ibuprofen sowie übliche Arzneimittel-Zusatzstoffe und/oder Arzneimittel-Trägerstoffe enthält und die dadurch gekennzeichnet ist, daß sie das Calcium-Ibuprofensalz und/oder das Calcium-S-(+)-Ibuprofensalz enthält.

Eine bevorzugte erfindungsgemäße Zubereitung enthält zusätzlich mindestens eine Verbindung aus der Gruppe Natrium-, Kalium-, Ammonium-Ibuprofensalz, Natrium-, Kalium-, Ammonium-S-(+)-Ibuprofensalz, Ibuprofen und S-(+)-Ibuprofen.

Gegenstand der Erfindung ist ebenfalls ein Verfahren zur Herstellung der oben genannten Zubereitung, das dadurch gekennzeichnet ist, daß Ibuprofen oder S-(+)-Ibuprofen oder ein Gemisch dieser Verbindungen, gegebenenfalls vermischt mit üblichen Arzneimittel-Zusatzstoffen und/oder Arzneimittel-Trägerstoffen, mit einer Calciumoxid-, Calciumhydroxid-, Calciumcarbonat-, Natriumhydroxid-, Kaliumhydroxid-, Ammoniumhydroxid-, Natriumcarbonat-, Natriumbicarbonat- oder Ammoniumbicarbonat-Lösung oder -Suspension behandelt wird, gegebenenfalls Arzneimittel-Zusatzstoffe und/oder -Trägerstoffe zugegeben werden, das erhaltene Gemisch in an sich bekannter Weise granuliert wird, gegebenenfalls getrocknet wird und gegebenenfalls nach Zugabe weiterer Arzneimittel-Hilfsstoffe und/oder -Trägerstoffe in an sich bekannter Weise in eine für die orale Verabreichung geeignete Form überführt wird.

Erfindungsgemäß wird das Ibuprofen und/oder das S-(+)-Ibuprofen in sein Calciumsalz überführt. Die Herstellung des Calciumsalzes erfolgt in an sich bekannter Weise. In dem Calciumsalz müssen Calcium und die Base nicht in stöchiometrischen Verhältnissen vorliegen. Die Säuren können in stöchiometrischem Überschuß vorhanden sein.

Gemäß einer bevorzugten erfindungsgemäßen Ausführungsform enthält das Calciumsalz zusätzlich ein Alkali- oder Ammoniumsalz. Erfindungsgemäß werden Alkali- oder Ammoniumsalze, vorzugsweise die Carbonate oder Hydrogencarbonate, verwendet. Als Alkalisalze oder -hydroxide werden bevorzugt die Natrium-, Kaliumsalze oder -hydroxide verwendet. Beispiele für solche Salze und Hydroxide sind Natriumcarbonat, Natriumhydrogencarbonat, Kaliumcarbonat, Kaliumhydrogencarbonat, Ammoniumcarbonat, Natriumhydroxid, Kaliumhydroxid, Ammoniumhydroxid und Ammoniumbicarbonat. Als Calciumverbindung können bei dem erfindungsgemäßen Verfahren das Calciumhydroxid, Calciumoxid und Calciumcarbonat verwendet werden. Von diesen sind Ammoniumhydroxid, Kaliumhydroxid und Calciumhydroxid besonders bevorzugt.

Die Ibuprofen-Calciumsalze sind die Grundsalze, die in dem erfindungsgemäßen Produkt vorhanden sind und die den Schmelzbereich unerwarteterweise hochdrücken und somit die Tablettierbarkeit verbessern. Die Ammonium- und die Alkalisalze verbessern je nach Anteil die Löslichkeit und steuern hiermit die Bioverfügbarkeit. Sie erhöhen jedoch die Hygroskopizität und außerdem die Klebrigkeit wieder.

Die Alkali-, Erdalkali- oder Ammoniumsalze oder -hydroxide werden in solchen Mengen verwendet, daß sie, bezogen auf das Ibuprofen, in 25 bis 110% der äquivalenten Menge vorhanden sind.

Sie müssen nicht unbedingt 100%ig äquivalent sein. Bereits 25% der berechneten Äquivalenzmenge können ausreichend sein, vorzugsweise genügen jedoch 40 bis 100%. Es sollen jedoch keine Zusatzmengen dieser Basen oder Salze eingesetzt werden, welche die berechneten äquivalenten Mengen um mehr als 10% übersteigen (insgesamt <110%). In diesen Fällen wird nämlich die Reaktion der wäßrigen Lösungen oder Suspensionen der erhaltenen Produkte zu alkalisch.

Erfindungsgemäß ist es bevorzugt, die Zusatzstoffe als wäßrige Lösung zu verwenden, wobei sich die Konzentration der wäßrigen Lösung nach der Löslichkeit der Zusatzstoffe richtet. Bei schwerlöslichen Zusatzstoffen kann man auch Suspensionen der Aufschwemmungen verwenden. Die Menge der Lösung oder Suspension richtet sich nach der Menge der Granulierflüssigkeit, welche zur Granulation benötigt wird, und kann von dem Fachmann leicht festgestellt werden. Wenn die Zusatzstoffe vollständig gelöst sind, reagieren sie schneller, die nichtgelösten reagieren nicht, so daß in diesem Fall Stoffgemische resultieren.

Unter den in bestimmter Weise abgestimmten Lösungen oder Aufschwemmungen von Alkali-, Erdalkali- oder Ammoniumhydroxiden, -carbonaten oder -hydrogencarbonaten sind vorzugsweise Zubereitungen bzw. Granulierflüssigkeiten zu verstehen. Es ist bevorzugt, daß die Menge an Zusatzstoff in Bezug auf Ibuprofen bemessen bzw. abgestimmt wird.

Durch die Salzbildung können auch die Löslichkeitseigenschaften der auf diese Weise gewonnenen Produkte gesteuert werden. Während nämlich die Alkali- und Ammoniumsalze der Ibuprofene gut in Wasser löslich sind, ist das Calciumsalz schlechter löslich. Durch gezieltes Mischen von Salzbildnern, die zu besser oder schlechter löslichen Salzen führen, lassen sich die Löslichkeiten bzw. die Lösungsgeschwindigkeiten der entstehenden Produkte in bestimmten Grenzen steuern. Durch die Steuerung der Löslichkeiten wird auch die Bioverfügbarkeit gesteuert. Ibuprofen-Calciumsalz ist schlechter löslich, weniger hygroskopisch, aber besser tablettierbar und granulierbar durch Erhöhung der Schmelztemperatur und Reduktion der Hygroskopizität.

Zu berücksichtigen ist, daß die 100%igen Alkalisalze (Na und K) nicht nur beträchtlich alkalischer reagieren, sondern auch sehr hygroskopisch sind. Deshalb empfiehlt sich ein 100%ig äquivalenter Einsatz bzw. die alleinige Verwendung von Alkalihydroxiden oder -salzen als Salzbildner weniger. Eine teilweise Neutralisation durch die Zugabe einer unteräquivalenten Menge von Alkalihydroxiden oder -carbonaten zu Ibuprofen oder S-(+)-Ibuprofen kann, wie bereits vorher erwähnt, bereits ausreichend puffern. Die Bildung von Mischsalzen aus Alkalihydroxiden oder -salzen, Ammoniumhydroxid oder -salzen mit Calciumhydroxid oder -carbonaten und den Ibuprofenen ist bevorzugt.

Gemäß einer bevorzugten erfindungsgemäßen Ausführungsform enthalten die Zubereitungen bis zu 40 Gew.-%, bezogen auf das Ibuprofen, einer niederen neutralen oder sauren Aminosäure. So werden gut tablettierbare Ibuprofen-Zubereitungen erhalten, wenn Ibuprofen oder S-(+)-Ibuprofen mit Puffer-Lösungen aus einer niederen Aminosäure und Alkali-, Erdalkali- oder Ammoniumhydroxiden oder -salzen als Granulierflüssigkeit in Mischern oder in der Wirbelschicht behandelt werden. Beispiele für niedere Aminosäuren sind Glycin Alanin, Valin, Asparaginsäure, Glutaminsäure, Prolin oder Hydroxyprolin. Die Aminosäuremenge kann bis zu 60 Gew.-%, berechnet auf das Ibuprofen, betragen. Vorzugsweise beträgt sie 15 bis 60 Gew.-%, besonders bevorzugt 30 bis 40 Gew.-%.
Eine zusätzliche Pufferung, insbesondere der stärkeren Basen, zum Beispiel NaOH und KOH, kann so durch Zusatz von neutralen oder sauren Aminosäuren erreicht werden. Ibuprofensäure reagiert leicht mit basischen Aminosäuren, zum Beispiel Lysin, nicht jedoch mit neutralen oder sauren Aminosäuren. Ibuprofen reagiert zum Beispiel nicht oder kaum mit Glycin (Aminoessigsäure) oder ähnlichen neutralen oder sauren Aminosäuren. Werden jedoch vorher die Na-, K-, NH₄- oder Ca-Salze von Glycin oder ähnlichen Aminosäuren gebildet, dann können diese gut mit Ibuprofen unter einer Mischsalzbildung reagieren. Es entstehen gut puffernde Gemische oder Komplexe. Bei Zusatz von Calcium ist die Löslichkeit der entstehenden Produkte nicht immer ausreichend.

Die Alkali-, Erdalkali- oder Ammoniumhydroxide oder -salze werden in denselben Mengen wie vorher bei den Zubereitungen ohne Aminosäuren als Puffer beschrieben eingesetzt.

Bei der Herstellung dieser Granulierflüssigkeiten wird zunächst das Glycin oder eine andere Aminosäure mit Alkali-, Erdalkali- oder Ammoniumhydroxid bzw. -salz oder Gemischen davon gemischt und in Wasser gelöst. Mit dieser Lösung wird dann Ibuprofen oder S-(+)-Ibuprofen in gleicher Weise wie vorher die Zubereitungen ohne Aminosäure-Puffer granuliert und sorgfältig getrocknet.

Mit den beschriebenen Mischer- und Wirbelschicht-Granulierprozessen, bei denen die entstehenden, mehr oder weniger salzartigen Ibuprofen-Produkte auch auf geeignete Träger, wie Cellulosepulver, mikrokristalline Cellulose oder Mannitol, aufgezogen werden können, um eine günstigere Dispersität zu erreichen, wird der Strukturaufbau derart verändert, daß sich diese Produkte nach Zusatz von üblichen Tablettier-Hilfsstoffen, wie Zerfallsbeschleunigern, Gleit- und Schmiermitteln, wesentlich rationeller und problemloser zu Tabletten verpressen lassen.

Die erfindungsgemäßen Produkte besitzen überraschenderweise verbesserte Tablettierungseigenschaften, vor allem im Hinblick auf Klebrigkeit, Hygroskopizität und Schmelzeigenschaften. Offensichtlich werden die verbesserten Tablettiereigenschaften durch Erhöhung der Schmelzbereichstemperaturen infolge vollständiger oder teilweiser Salzbildung während des Granulierprozesses, je nach Art und Menge der eingesetzten Alkalien oder ihrer Carbonate, herbeigeführt.

Durch Zumischung von Alkali- und/oder NH₄-Hydroxiden oder -Salzen werden die Löslichkeiten und hiermit die Bioverfügbarkeiten wesentlich verbessert. Neben verbesserten Tablettiereigenschaften und einer in bestimmter Weise gesteuerten Lösungsgeschwindigkeit der Ibuprofene (Arzneistofffreigabe/Bioverfügbarkeit) kann diesen gepufferten Zubereitungen auch noch eine verbesserte Verträglichkeit zugeschrieben werden, wie dies von "Buffered Aspirin" her bekannt ist. Acetylsalicylsäure kann Schleimhäute reizen, und durch Puffer-Zusatz können diese Erscheinungen reduziert werden (W. Hangarter: Die Salicylsäure und ihre Abkömmlinge, F.K. Schattauer Verlag, Stuttgart, New York, 1974, S. 354/355).

Die erfindungsgemäßen Zubereitungen liegen in einer für die orale Verabreichung geeigneten Form, beispielsweise in Form von Tabletten, Dragees, Filmtabletten, Kapseln, als Granulat oder in Form von Tabletten, Dragees, Filmtabletten, Kapseln, Granulat mit verzögerter Wirkung vor. Zur Herstellung von Arzneimittelzubereitungen mit verzögerter Wirkstoffabgabe ist es bevorzugt, die reinen Calciumsalze, d.h. ohne Zugabe von Alkali- oder Ammoniumsalzen, erforderlichenfalls zusammen mit üblichen retardierenden Zusatz- oder Hilfsstoffen, zum Beispiel hydriertes Rizinusöl oder Carboxymethylcellulose, zu verwenden. Dadurch wird die Bioverfügbarkeit in bestimmter Weise retardiert. Es ist möglich, die reinen oder separat hergestellten Ibuprofensalze in die Arzneimittelzubereitungen einzuarbeiten oder die Herstellung der Zubereitungen so vorzunehmen, daß die Salze dabei entstehen. Als Arzneimittelzubereitungen sind besonders die festen geeignet, zum Beispiel Granulate, Pellets, Tabletten, Filmtabletten und Dragees (film- oder zuckerumhüllte Formen) und Kapseln. Als Salzmischung werden je nach gewünschter Freisetzungs- bzw. Auflösungsgeschwindigkeit der Zubereitung bestimmte Verhältnisse zwischen löslichen und unlöslichen oder gut und schlecht löslichen Salzen eingesetzt. Die Salze entstehen, wie oben ausgeführt wurde, durch Reaktion des Ibuprofens mit CaO, Ca(OH)₂, CaCO₃, NaOH, KOH, NH₄OH, Na₂CO₃, NaHCO₃, K₂CO₃, KHCO₃, (NH₄)₂CO₃, NH₄HCO₃, also mit Basen oder mit basischen Salzen.

Die folgenden Beispiele erläutern die Erfindung ohne sie zu beschränken.

### Beispiel 1

200,0 g Ibuprofen werden in einem Wirbelschichtgranulator mit 126,0 g Mannitol und 10,0 g Polyvidon gemischt und dann mit einer Granulierflüssigkeit aus Kalkmilch (28,5 g Ca(OH)₂ und 100,0 g Wasser) und 42,5 g Ammoniaklösung 10%ig besprüht. Gleichzeitig wird mit Warmluft von 70 bis 120°C getrocknet. Sobald das Granulat den vorgeschriebenen Trocknungsgrad erreicht hat, wird es mit nicht erwärmter Luft kaltgeblasen, durch ein 1 mm-Sieb geschlagen und nach Zusatz von 30,0 g Weizenstärke, 7,0 g Ac-Di-Sol (quervernetzte Natrium-Carboxymethylcellulose) und 3,0 g Calciumarachinat als Spreng-, Gleit- und Schmiermittel in üblicher Weise tablettiert.

### Beispiel 2

200,0 g S-(+)-Ibuprofen werden mit 28,5 g Ca(OH)₂ gemischt und anschließend mit 70,0 g Kalilauge 20%ig befeuchtet. Nach gleichmäßiger Befeuchtung werden 80,0 g Maisstärke zugesetzt und noch ca. 80,0 bis 100,0 g Wasser portionsweise eingeknetet, bis eine feuchtplastische granulierbare Masse entstanden ist. Diese wird durch ein 3 mm-Sieb feuchtgranuliert und in einem Wirbelschichttrockner bei 40°C getrocknet. Nach Erreichen des gewünschten Trocknungsgrades wird durch ein 1 mm-Sieb gesiebt. Zuletzt werden 25,0 g Maisstärke und 10,0 g mit 10% Stearinsäure überzogenes Talkum als äußere Phase zugemischt und tablettiert.

### Beispiel 3

75,0 g Glycin werden in 180,0 g Wasser gelöst und anschießend werden in dieser Lösung 38,0 g Ca(OH)₂ unter Rühren und Erwärmen aufgelöst. Mit dieser Lösung wird eine Mischung aus 200,0 g Ibuprofen, 50,0 g mikrokristalliner Cellulose und 10,0 g hydrolysierter Gelatine angeknetet, erforderlichenfalls wird noch etwas Wasser zugesetzt, bis eine feuchtplastische, granulierbare Masse entstanden ist. Diese Masse wird durch ein 3 mm-Sieb feuchtgranuliert. Nach der Trocknung bei 60°C und Siebung durch ein 0,8 mm-Sieb werden 30,0 g Weizenstärke, 7,0 g quervernetztes Polyvidon und 3,0 g Calciumarachinat als Zerfallsbeschleuniger, Gleit- und Schmiermittel zugesetzt und dann wird in üblicher Weise tablettiert.

### Beispiel 4

75,0 g Glycin werden in 130,0 bis 150,0 g Wasser gelöst, 70,0 g Kalilauge 20%ig werden zugesetzt. In dieser Lösung werden unter Erwärmung 28,5 g Ca(OH)₂ gelöst. Mit dieser Lösung wird in einem Wirbelschichtgranulator eine Mischung aus 200,0 g S-(+)-Ibuprofen und 100,0 g mikrokristalline Cellulose besprüht und anschließend mit Warmluft von 60°C getrocknet. Sobald der vorgeschriebene Trocknungsgrad erreicht ist, wird mit nicht erwärmter, trockener Luft kaltgeblasen, das Granulat aus der Apparatur entnommen, gesiebt, die üblichen Spreng-, Gleit- und Schmiermittel zugemischt und tablettiert.

## Patentansprüche

1. Zubereitung mit verbesserter Tablettierbarkeit, die Ibuprofen und/oder S-(+)-Ibuprofen sowie übliche Arzneimittel-Zusatzstoffe und/oder Arzneimittel-Trägerstoffe enthält, dadurch **gekennzeichnet,** daß sie das Calcium-Ibuprofensalz und/oder das Calcium-S-(+)-Ibuprofensalz enthält.

2. Zubereitung nach Anspruch 1, dadurch **gekennzeichnet,** daß sie zusätzlich mindestens eine Verbindung aus der Gruppe Natrium-, Kalium-, Ammonium-Ibuprofensalz, Natrium-, Kalium-, Ammonium-S-(+)-Ibuprofensalz, Ibuprofen und S-(+)-Ibuprofen enthält.

3. Zubereitung nach Anspruch 1 oder 2, dadurch **gekennzeichnet,** daß sie
(a) 50 bis 100 Gew. -%
Calcium-Ibuprofensalz und/oder Calcium-S-(+)-Ibuprofensalz,
(b) 0 bis 50 Gew.-%
Natrium-, Kalium-, Ammonium-Ibuprofensalz, Natrium-, Kalium-, Ammonium-S-(+)-Ibuprofensalz, Ibuprofen, S-(+)-Ibuprofen
enthält, bezogen auf das Gemisch aus den Bestandteilen der Gruppe (a) und der Gruppe (b).

4. Zubereitung nach einem der Ansprüche 1, 2 oder 3, dadurch **gekennzeichnet,** daß sie 15 bis 60% des Äquivalentgewichtes, bezogen auf den Wirkstoff Ibuprofen und/oder S-(+)-Ibuprofen, einer neutralen oder sauren Aminosäure enthält.

5. Zubereitung nach Anspruch 4, dadurch **gekennzeichnet,** daß sie als Aminosäure Glycin, Alanin, Valin, Asparaginsäure, Glutaminsäure, Prolin oder Hydroxyprolin enthält.

6. Zubereitung nach mindestens einem der Ansprüche 1 bis 5, dadurch **gekennzeichnet,** daß sie in einer für die orale Verabreichung geeigneten Form vorliegt.

7. Zubereitung nach Anspruch 6, dadurch **gekennzeichnet,** daß sie in Form von Tabletten, Dragees, Filmtabletten, Kapseln, als Granulat oder in Form von Tabletten, Dragees, Filmtabletten, Kapseln, Granulat mit verzögerter Wirkung vorliegt.

8. Verfahren zur Herstellung der Zubereitung nach mindestens einem der Ansprüche 1 bis 7, dadurch **gekennzeichnet,** daß Ibuprofen oder S-(+)-Ibuprofen oder ein Gemisch dieser Verbindungen, gegebenenfalls vermischt mit üblichen Arzneimittel-Zusatzstoffen und/oder Arzneimittel-Trägerstoffen, mit einer Calciumoxid-, Calciumhydroxid-, oder Calciumcarbonat-, Lösung und gegebenenfalls mit einer Natriumhydroxid-, Kaliumhydroxid-, Ammoniumhydroxid-, Natriumcarbonat-, Natriumbicarbonat-, Kaliumcarbonat-, Kaliumbicarbonat-, Ammoniumcarbonat- oder Ammoniumbicarbonat-Lösung oder -Suspension behandelt wird, gegebenenfalls Arzneimittel-Zusatzstoffe und/oder -Trägerstoffe zugegeben werden, das erhaltene Gemisch in an sich bekannter Weise granuliert wird, gegebenenfalls getrocknet wird und gegebenenfalls nach Zugabe weiterer Arzneimittel-Hilfsstoffe und/oder -Trägerstoffe in an sich bekannter Weise in eine für die orale Verabreichung geeignete Form überführt wird.

9. Verfahren nach Anspruch 8, dadurch **gekennzeichnet,** daß die Calcium-, Alkali- oder Ammoniumverbindung in der 25- bis 110% der äquivalenten Menge, bezogen auf den Wirkstoff, verwendet wird.

10. Verfahren nach einem der Ansprüche 8 oder 9, dadurch **gekennzeichnet,** daß bei irgendeiner beliebigen Stufe des Verfahrens eine neutrale oder saure Aminosäure in einer Menge von 50 Gew.-% auf den Wirkstoff in fester Form, als Lösung oder als Suspension zugegeben wird.

11. Verfahren nach Anspruch 10, dadurch **gekennzeichnet,** daß die niedere Aminosäure der Granulierflüssigkeit zugegeben wird.

12. Verfahren nach mindestens einem der Ansprüche 8 bis 11, dadurch **gekennzeichnet,** daß als eine für die orale Verabreichung geeignete Form Tabletten, Dragees, Filmtabletten, Kapseln, ein Granulat oder Tabletten, Dragees, Filmtabletten, Kapseln, ein Granulat mit verzögerter Wirkung hergestellt wird.

## Claims

1. A preparation having improved tabletting capacity, which contains ibuprofen and/or S-(+)-ibuprofen as well as customary drug additives and/or drug vehicles, characterised in that it contains the calcium salt of ibuprofen and/or the calcium salt of S-(+)-ibuprofen.

2. A preparation according to claim 1, characterised in that it additionally contains at least one compound from the group comprising the sodium, potassium or ammonium salt of ibuprofen, the sodium, potassium or ammonium salt of S-(+)-ibuprofen, ibuprofen and S-(+)-ibuprofen.

3. A preparation according to claim 1 or 2, characterised in that it contains
(a) 50 to 100 % by weight
of the calcium salt of ibuprofen and/or the calcium salt of S-(+)-ibuprofen, and
(b) 0 to 50 % by weight
of the sodium, potassium or ammonium salt of ibuprofen, the sodium, potassium or ammonium salt of S-(+)-ibuprofen, ibuprofen or S-(+)-ibuprofen,
with respect to the mixture of constituents of group (a) and of group (b).

4. A preparation according to any one of claims 1,2 or 3, characterised in that it contains 15 to 60 % of the equivalent weight of a neutral or acidic amino acid with respect to the active ingredient ibuprofen and/or S-(+)-ibuprofen.

5. A preparation according to claim 4, characterised in that it contains glycine, alanine, valine, aspartic acid, glutamic acid, proline or hydroxyproline as the amino acid.

6. A preparation according to at least one of claims 1 to 5, characterised in that it exists in a form suitable for oral administration.

7. A preparation according to claim 6, characterised in that it exists in the form of tablets, dragees, coated tablets, capsules or as a granular material, or in the form of tablets, dragees, coated tablets, capsules or granular material with a delayed action.

8. A process for producing the preparation according to at least one of claims 1 to 7, characterised in that ibuprofen or S-(+)-ibuprofen or a mixture of these compounds, optionally mixed with other customary drug additives and/or drug vehicles, is treated with a solution of calcium oxide, calcium hydroxide or calcium carbonate and optionally with a solution or suspension of sodium hydroxide, potassium hydroxide, ammonium hydroxide, sodium carbonate, sodium bicarbonate, potassium carbonate, potassium bicarbonate, ammonium carbonate or ammonium bicarbonate, drug additives and/or drug vehicles are optionally added, the mixture obtained is granulated in a manner known in the art, optionally dried, and is converted in a manner known in the art, optionally after the addition of further drug auxiliary materials and/or drug vehicles, into a form suitable for oral administration.

9. A process according to claim 8, characterised in that the calcium, alkali or ammonium compound is used in 25 to 110 times the equivalent amount with respect to the active ingredient.

10. A process according to either one of claims 8 or 9, characterised in that at any arbitrary stage in the process a neutral or acidic amino acid is added in solid form, as a solution or as a suspension to the active ingredient in an amount of 50 % by weight.

11. A process according to claim 10, characterised in that the lower amino acids are added to the granulation liquid.

12. A process according to at least one of claims 8 to 11, characterised in that tablets, dragees, coated tablets, capsules or a granular material, or tablets, dragees, coated tablets, capsules or a granular material with a delayed action are produced as a form suitable for oral administration.

## Revendications

1. Préparation ayant une aptitude améliorée à être transformée en comprimés, contenant de l'ibuprofène et/ou du S-(+)-ibuprofène, ainsi que des additifs usuels pour des médicaments et/ou des véhicules pour des médicaments, caractérisée en ce qu'elle contient le sel de calcium de l'ibuprofène et/ou le sel de calcium du S-(+)-ibuprofène.

2. Préparation selon la revendication 1, caractérisée en ce qu'elle contient, en outre, au moins un composé choisi dans l'ensemble constitué par le sel de sodium, le sel de potassium et le sel d'ammonium de l'ibuprofène, ainsi que le sel de sodium, le sel de potassium et le sel d'ammonium du S-(+)-ibuprofène.

3. Préparation selon la revendication 1 ou 2, caractérisée en ce qu'elle contient
(a) 50 à 100 % en poids de sel de calcium de l'ibuprofène et/ou de sel de calcium du S-(+)-ibuprofène,
(b) 0 à 50 % en poids de sel de sodium, de sel de potassium ou de sel d'ammonium de l'ibuprofène, de sel de sodium, de sel de potassium ou de sel d'ammonium du S-(+)-ibuprofène,
par rapport au mélange des constituants du groupe (a) et du groupe (b).

4. Préparation selon l'une des revendications 1, 2 ou 3, caractérisée en ce qu'elle contient de 15 à 60 % du poids d'équivalents, par rapport au principe actif ibuprofène et/ou S-(+)-ibuprofène, d'un amino-acide neutre ou acide.

5. Préparation selon la revendication 4, caractérisée en ce qu'elle contient, comme amino-acide, la glycine, l'alanine, la valine, l'acide aspartique, l'acide glutamique, la proline ou l'hydroxyproline.

6. Préparation selon au moins l'une des revendications 1 à 5, caractérisée en ce qu'elle a une forme appropriée à une administration orale.

7. Préparation selon la revendication 6, caractérisée en ce qu'elle se présente sous la forme de comprimés, de dragées, de comprimés enrobés, de capsules ou de granules, ou sous la forme de comprimés, de dragées, de comprimés enrobés, de capsules ou de granules à effet retard.

8. Procédé d'obtention de la préparation selon au moins l'une des revendications 1 à 7, caractérisé en ce qu'on traite l'ibuprofène ou le S-(+)-ibuprofène ou un mélange de ces composés, éventuellement mélangés avec des additifs usuels pour médicaments et/ou des véhicules usuels pour médicaments, avec une solution d'oxyde de calcium, d'hydroxyde de calcium ou de carbonate de calcium, et éventuellement avec une solution ou une suspension d'hydroxyde de sodium, d'hydroxyde de potassium, d'hydroxyde d'ammonium, de carbonate de sodium, de bicarbonate de sodium, de carbonate de potassium, de bicarbonate de potassium, de carbonate d'ammonium ou de bicarbonate d'ammonium, on ajoute éventuellement des additifs pour médicaments et/ou des véhicules pour médicaments, on transforme le mélange obtenu en granules, d'une manière connue en soi, éventuellement on sèche ces granules, et après l'addition éventuelle d'autres adjuvants et/ou véhicules pour médicaments, on les transforme, d'une manière connue en soi, en une forme appropriée à une administration orale.

9. Procédé selon la revendication 8, caractérisé en ce qu'on utilise le composé du calcium, de l'alcali ou de l'ammonium en une quantité d'équivalent valant de 25 à 110 fois la quantité de principe actif.

10. Procédé selon la revendication 8 ou 9, caractérisé en ce que, pendant n'importe quelle étape désirée du procédé, on ajoute un amino-acide neutre ou acide, en une quantité de 50 % en poids par rapport au principe actif, à l'état solide ou sous la forme d une solution ou d'une suspension.

11. Procédé selon la revendication 10, caractérisé en ce qu'on ajoute l'amino-acide inférieur au liquide à transformer en granules.

12. Procédé selon au moins l'une des revendications 8 à 11, caractérisé en ce qu'on prépare, sous une forme appropriée à une administration orale, des comprimés, des dragées, des comprimés enrobés, des capsules ou des granules, ou des comprimés, des dragées, des comprimés enrobés, des capsules ou des granules à effet retard.
